# FASCICULE DE BREVET EUROPEEN

(11) **EP 0 519 846 B1**
(45) Date de publication et mention de la délivrance du brevet: **13.12.1995**
(21) Numéro de dépôt: 92420206.2
(22) Date de dépôt: 16.06.1992
(51) Int. Cl.: A63B 23/18

(54) **Méthode de musculation abdominale et dispositif permettant d'assurer la mise en oeuvre de cette méthode**
Verfahren und Vorrichtung zur abdominalen Muskelausbildung
Method and device for abdominal musculation

(30) Priorité: 18.06.1991 FR 9107743
(43) Date de publication de la demande: 23.12.1992
(73) Titulaire: Guillarme, Luc, F-71100 Chalon sur Saone (FR)
(72) Inventeur: Guillarme, Luc, F-71100 Chalon sur Saone (FR)
(74) Mandataire: Laurent, Michel

(56) Documents cités:
- EP-A- 0 372 148
- DE-C- 74 076
- FR-A- 1 495 348

## Description

L'invention concerne une méthode de musculation abdominale, destinée à éduquer le fonctionnement de l'ensemble des muscles abdominaux.

Elle concerne également un dispositif destiné à mettre en oeuvre et à favoriser l'action de cette méthode de musculation.

Par muscles abdominaux, on entend essentiellement les muscles grands droits, les muscles obliques et le transverse : ils constituent la sangle abdominale. De leur tonicité, dépend de manière connue le bon fonctionnement de certains organes, que ce soit ceux liés au processus de la digestion ou ceux concernant la miction, la défécation, l'expulsion ou la contention. Ainsi, l'invention est-elle dirigée vers une méthode de récupération de la sangle abdominale, telle que définie ci-dessus.

De manière traditionnelle, les méthodes de musculation abdominale consistent à provoquer une contraction des muscles abdominaux, mais qu'il s'agisse de mouvements du tronc par rapport aux membres inférieurs, ou des membres inférieurs par rapport au tronc (flexion, extension, rotation), la participation du diaphragme au cours de ces mouvements contribue à augmenter la pression abdominale.

Si certes, ces méthodes traditionnelles améliorent la tonicité des muscles abdominaux, en revanche, elles ne permettent pas d'aboutir à une détente diaphragmatique et, corrélativement, à un confort "pelvien" de l'individu.

L'invention vise une méthode de musculation abdominale visant à corréler le mouvement proprement dit des abdominaux avec le mouvement du diaphragme.

Cette méthode de musculation abdominale est définie dans la revendication 1 et consiste à expirer l'air des poumons en deux phases continues : une première phase d'expiration thoracique sans prise d'air préalable, suivie d'une seconde phase d'expiration abdominale, consistant en une contraction isotonique des muscles abdominaux, l'exsufflation étant en outre favorisée par le maintien en position semi-ouverte des deux mâchoires.

En d'autres termes, l'invention consiste non plus à opérer une contraction isométrique des muscles abdominaux et un blocage corollaire du diaphragme, mais à procéder à une méthode respiratoire donc associée au mouvement du diaphragme, et à induire sa remontée maximum par réalisation d'une exsufflation complète.

Selon une caractéristique fondamentale de l'invention, le maintien en position semi-ouverte des deux machoires est assuré par un embout d'exsufflation cylindrique défini dans la revendication 3. Celui-ci est maintenu entre les dents et les lèvres et présente un diamètre interne compris entre cinq (5) et dix (10) millimètres. Il est muni d'une saillie annulaire latérale afin d'éviter tout risque de pénétration de l'embout trop profondément dans la bouche de l'individu.

Selon une autre caractéristique essentielle de l'invention, la méthode s'accompagne d'une électro-neuro stimulation abdominale ou périnéale, réalisée au moyen d'un courant électrique sous forme d'impulsions, délivrées au niveau des muscles abdomioaux ou de la cavité périnéale, la fréquence et les caractéristiques des impulsions étant bien déterminées. Le courant délivré est un courant tétanisant trophique ayant un effet polyvalent tant sur l'abdomen pour une musculation abdominale, que pour une rééducation périnéale spécifique. Un dispositif de mise en oeuvre de cette méthode est défini dans la revendication 5.

La manière dont l'invention peut-être réalisée et les avantages qui en découlent ressortiront mieux de l'exemple de réalisation qui suit donné à titre indicatif et non limitatif à l'appui des figures annexées.

La figure 1 est une représentation schématique en coupe longitudinale de l'embout d'exsufflation conforme à l'invention.

La figure 2 est une vue en coupe transversale dudit embout.

Selon l'invention, la méthode de musculation abdominale consiste à opérer une pluralité de cycles d'expiration en deux phases continues, la première phase étant une phase d'expiration thoracique complète suivie d'une seconde phase dite d'expiration abdominale complète, consistant en la contraction isotonique des muscles abdominaux, en d'autres termes, la rentrée en direction de l'intérieur du corps de ces muscles, provoquant une remontée du diaphragme vers le haut, afin d'induire une expiration thoracique totale.

Ces deux phases d'expiration thoracique puis abdominale sont améliorées par la mise en place entre les lèvres de l'individu concerné d'un embout d'exsufflation cylindrique (1).

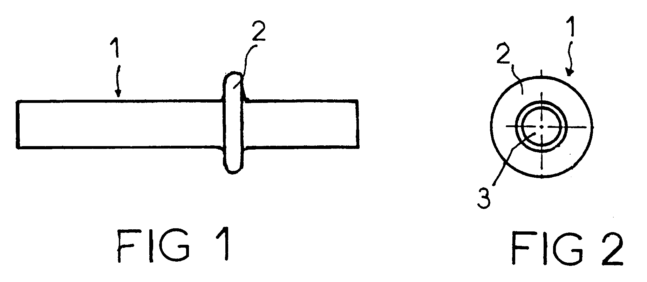

On a représenté sur la figure 1 une forme de réalisation d'un tel embout d'exsufflation. Celui est réalisé en un matériau approprié, tel que notamment en carton, matière plastique, etc... . Il présente une longueur typique de 80 millimètres et surtout un diamètre interne (3) compris entre 5 et 10 millimètres. De la sorte, l'individu en cours d'exsufflation est induit à ne pas bloquer cette respiration en fermant la bouche et notamment les lèvres, compte-tenu de la présence de cet embout.

Il est muni d'une saillie annulaire (2), destinée à venir en contact avec la face labiale des dents antérieures, c'est à dire des incisives, de telle sorte à ne pas risquer soit d'avaler l'embout, soit une pénétration trop profonde de celui-ci à l'intérieur de la bouche de l'individu. De fait, cette saillie annulaire (2) est positionnée entre le tiers et la moitié de la longueur totale dudit embout (1).

On observe que la détente diaphragmatique obtenue, induit une mobilisation des différents viscères vers le haut. Ce résultat fondamental peut avoir de nombreuses applications, notamment dans le domaine thérapeutique, mais également dans celui de la réadaptation.

Selon l'invention, cette méthode de musculation est associée à une électro-neuro stimulation. Celle-ci consiste à produire des contractions musculaires par la pénétration transcutanée de courants électriques spécifiques au moyen d'éléctrodes de surface, une éléctrode positive est placée sur la partie inférieure des muscles grands droits et une électrode négative est placée sur le muscle transverse de l'abdomen.

La fréquence préférentielle est de 70 hertz (Hz). La largeur d'impulsion est de O,4 milliseconde. Si l'on choisit un mode de déclenchement automatique synchrone, consistant à stimuler de manière synergique les muscles abdominaux droits et gauches, la durée de travail, d'environ 4 secondes, comporte un temps de montée de courant de 1,5 seconde, puis un plateau à intensité constante de 2 secondes, suivi d'un temps de descente de 0,5 seconde.

C'est durant cette période de 4 secondes que l'individu effectue une expiration complète thoracique, puis abdominale, et ce, sans prise d'air préalable, et un embout d'exsufflation placé entre les dents et les lèvres. L'intensité est variable (0 à 100 milliampères) et dépendante de la trophicité de l'individu et de son impédance. Le temps de repos est de 12 secondes, le cycle travail-repos étant donc de 16 secondes.

On peut avantageusement prévoir un signal lumineux et/ou sonore précédant d'une seconde l'arrivée du courant, afin de prévenir l'individu de l'imminence d'une phase de travail, le préparer à ne pas prendre de l'air avant l'effort d'expiration, et surtout éviter l'attente crispante de l'effet de contraction créé par le stimulateur.

Dans ce cas, l'individu déclenche manuellement l'appareil de stimulation au moment de l'effort d'expiration thoracique puis abdominale, et maintient le passage du courant le temps de ses possibilités expiratoires. Le rythme de travail-repos reste libre et est fonction de la fatigabilité de l'individu. De fait l'électro-stimulateur comprend des paramètres fixes, tels que notamment la fréquence : 70 Hz, la largeur d'impulsion : 0,4 millisecondes, le temps de montée du courant : 0,8 seconde) mais également des paramètres modulables : le temps de travail réglé et maintenu au moyen d'un déclencheur situé sur l'appareil, potentiomètre permettant d'augmenter l'intensité du courant de 0 à 100 milliampères.

On peut en outre relier l'embout d'exsufflation à l'éléctro-neuro-stimulateur au moyen d'un tube flexible, et c'est le début de l'expiration qui déclenche la stimulation électrique. De la sorte, on aboutit à une meilleure corrélation entre le travail proprement dit et la stimulation.

Dans une variante de l'invention, on peut préférer à une électro-stimulation abdominale, une électro-stimulation périnéale. Dans ce cas, on utilise des électrodes auto-adhésives, ou mieux, une sonde endocavitaire, positionnée au contact de la cavité périnéale. Le cycle de travail reste équivalent, sinon identique à celui décrit précédemment.

Cette méthode de musculation abdominale s'avère particulièrement adaptée pour la recherche d'une détente diaphragmatique, pour l'amélioration de la statique vertébrale, et reste la base de tous les traitements cherchant à récupérer le bon fonctionnement des organes pelviens.

## Revendications

1. Méthode de musculation abdominale caractérisée :
- en ce qu'elle consiste à expirer l'air contenu dans les poumons en deux phases continues, une première phase d'expiration thoracique sans prise d'air préalable, suivie d'une seconde phase d'expiration abdominale, consistant en une contraction isotonique des muscles abdominaux, l'exsufflation étant en outre favorisée par le maintien en position semi-ouverte des deux mâchoires, assuré par un embout d'exsufflation cylindrique (1), inséré entre les dents et les lèvres ;
- et en ce qu'elle s,accompagne d'une électro-neuro-stimulation abdominale ou périnéale, réalisée au moyen d'un courant électrique sous forme d'impulsions, délivrées au niveau des muscles abdominaux ou de la cavité périnéale, la fréquence et les caractéristiques des impulsions étant bien déterminées.

2. Méthode selon la revendication 1, caractérisée en ce que l'éléctro-neuro-stimulation est corrélée avec l'expiration détectée de l'individu.

3. Embout d'exsufflation pour la mise en oeuvre de la méthode selon la revendication 1, caractérisé en ce qu'il est constitué par un cylindre (1) dont le diamètre interne est compris entre cinq et dix millimètres, ledit cylindre étant muni d'une saillie annulaire (2) latérale, destinée à venir s'appuyer sur la surface labiale des dents antérieures de l'individu.

4. Embout d'exsufflation selon la revendication 3, caractérisé en ce que la saillie annulaire latérale (2) est positionnée entre le tiers et la moitié de la longueur totale dudit embout (1).

5. Dispositif pour la mise en oeuvre de la méthode selon la revendication 2, caractérisé en ce qu'il comprend :
- un embout d'exsufflation constitué d'un cylindre (1), dont le diamètre interne est compris entre cinq et dix millimètres, et muni d'une saillie annulaire latérale (2) positionnée entre le tiers et la moitié de la longueur totale dudit embout,
- un appareil d'électro-neuro-stimulation constitué par un organe délivreur d'impulsions électriques, l'extrémité libre dudit embout d'exsufflation étant connectée à un tube souple, relié audit appareil d'électro-neuro-stimulation, afin d'induire, lors des phases d'expiration, le déclenchement des impulsions électriques.

## Claims

1. Method for abdominal musculation characterised:
- in that it involves breathing air contained in the lungs in two continuous phases, a first thoracic expiration phase without previously taking in air, followed by a second abdominal expiration phase consisting of an isotonic contraction of the abdominal muscles, breathing out also being promoted by maintaining the upper and lower jaws in a semi-open position by a cylindrical exhalation mouthpiece (1) inserted between the teeth and the lips;
- and in that it is accompanied by electrical abdominal or perineal nerve stimulation by means of an electric current in the form of pulses applied to the abdominal muscles or the perineal cavity, the frequency and characteristics of the pulses being clearly defined.

2. Method as claimed in claim 1, characterised in that electrical nerve stimulation is correlated with detected expiration of the individual.

3. Exhalation mouthpiece for using the method as claimed in claim 1, characterised in that it consists of a cylinder (1) of which the inside diameter is between five and ten millimetres, said cylinder being fitted with a laterally projecting ring (2) intended to rest against the labial surface of the anterior teeth of the individual.

4. Exhalation mouthpiece as claimed in claim 3, characterised in that laterally projecting ring (2) is positioned between one third and one half along the total length of said mouthpiece (1).

5. Device for using the method as claimed in claim 2, characterised in that it comprises:
- an exhalation mouthpiece consisting of a cylinder (1) of which the inside diameter is between five and ten millimetres and fitted with a laterally projecting ring (2) positioned between one third and one half along the total length of said mouthpiece,
- apparatus for electrical nerve stimulation consisting of a unit that delivers electrical pulses, the free end of said exhalation mouthpiece being connected to a flexible tube linked to said electrical nerve stimulation apparatus in order to cause, during expiration phases, the triggering of electrical pulses.

## Patentansprüche

1. Verfahren zur Bauchmuskelausbildung, dadurch gekennzeichnet, daß:
- die in den Lungen befindliche Luft in zwei kontinuierlichen Phasen ausgeamtet wird - eine erste Phase der Brustausatmung ohne vorheriges Einatmen, gefolgt von einer zweiten Phase der Bauchausatmung bestehend aus einer isotonischen Kontraktion der Bauchmuskeln, wobei die Exsufflation außerdem durch die Aufrechterhaltung einer halboffenen Stellung von Ober- und Unterkiefer begünstigt wird, die durch ein zwischen Zähnen und Lippen eingeschobenes zylindrisches Ausblasstück (1) gewährleistet wird;
- und dadurch gekennzeichnet, daß es durch eine abdominale oder perineale Elektro-Neuro-Stimulation in Form von Stromimpulsen im Bereich der Bauchmuskeln oder der Dammhöhle begleitet wird, wobei Frequenz und Merkmale der Impulse klar definiert sind.

2. Verfahren nach Anspruch 1, dadurch gekennzeichnet, daß die Elektro-Neuro-Stimulation mit der erfaßten Ausatmung der Behandlungsperson korreliert.

3. Ausblasstück für die Durchführung des Verfahrens nach Anspruch 1, dadurch gekennzeichnet, daß es aus einem Zylinder (1) besteht, dessen Innendurchmesser zwischen fünf und zehn Millimeter beträgt, wobei der Zylinder einen seitlichen ringartigen Vorsprung (2) besitzt, der an der labialen Fläche der vorderen Zähne der Behandlungsperson anliegen soll.

4. Ausblasstück nach Anspruch 3, dadurch gekennzeichnet, daß der seitliche ringartige Vorsprung (2) zwischen einem Drittel und der Hälfte der Gesamtlänge des Ausblasstücks positioniert ist.

5. Vorrichtung für die Durchführung des Verfahrens nach Anspruch 2, dadurch gekennzeichnet, daß sie folgende Teile umfaßt:
- ein Ausblasstück bestehend aus einem Zylinder (1), dessen Innendurchmesser zwischen fünf und zehn Millimeter beträgt, und der einen seitlichen ringartigen Vorsprung (2) besitzt, der zwischen einem Drittel und der Hälfte der Gesamtlänge des Ausblasstücks positioniert ist,
- ein Gerät für die Elektro-Neuro-Stimulation bestehend aus einem Organ für die Abgabe von elektrischen Impulsen, wobei das freie Ende des Ausblasstücks an ein flexibles Rohr angeschlossen ist, das mit dem besagten Gerät für die Elektro-Neuro-Stimulation in Verbindung steht, um bei den Ausatmungsphasen das Auslösen der elektrischen Impulse zu bewirken.
